(19) Europäisches Patentamt
European Patent Office
Office européen des brevets

(11) Veröffentlichungsnummer: **0 001 801**
**B1**

(12) # EUROPÄISCHE PATENTSCHRIFT

(21) Anmeldenummer: **78101212.5**

(22) Anmeldetag: **24.10.78**

(51) Int. Cl.³: **C 09 B 49/10,**
C 07 D 279/20,
C 07 D 279/34, D 06 P 1/30

(54) **Kondensationsprodukt aus Phenothiazin und p-Nitrosophenol, Verfahren zur Herstellung des Kondensationsprodukts, Verfahren zur Herstellung von Schwefelfarbstoffen unter Verwendung des Kondensationsprodukts und die hergestellten Schwefelfarbstoffe**

(30) Priorität: **29.10.77 DE 2748744**

(43) Veröffentlichungstag der Anmeldung:
**16.05.79 Patentblatt 79/10**

(45) Bekanntmachung des Hinweises auf die Patenterteilung:
**26.11.80 Patentblatt 80/24**

(84) Benannte Vertragsstaaten:
**BE CH DE FR GB NL**

(56) Entgegenhaltungen:
**US - A - 1 867 863**

**THE JOURNAL OF THE CHEMICAL SOCIETY
Vol. 125, 1924, London
SIKHIBHUSHAN DUTT "Dyes derived from Carbazole and Thiodiphenylamine"
Seiten 802—807**

**K. VENKATARAMAN "The chemistry of synthetic dyes"
Vol. II, 1952, New York, USA
Academic Press
Seiten 1087 und 1088**

(73) Patentinhaber: **CASSELLA Aktiengesellschaft
Hanauer Landstrasse 526
D - 6000 Frankfurt am Main 61 (DE)**

(72) Erfinder: **Nagl, Gert, Dr.
Hanauer Landstrasse 497
D - 6000 Frankfurt am Main 61 (DE)
Ribka, Joachim, Dr.
Rügener Strasse 4
D - 6050 Offenbach am Main-Bürgel (DE)
Dickmanns, Heinz
Lauterbacher Strasse 8
D - 6000 Frankfurt am Main 61 (DE)
Gotsmann, Ulrich, Dr.
Fritz-Schubert-Ring 45
D - 6000 Bergen-Enkheim (DE)**

(74) Vertreter: **Urbach, Hans-Georg, Dr., et al
Hanauer Landstrasse 526
D - 6000 Frankfurt am Main 61 (DE)**

**EP 0 001 801 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Kondensationsprodukt aus Phenothiazin und p-Nitrosophenol, Verfahren zur Herstellung des Kondensationsprodukts, Verfahren zur Herstellung von Schwefelfarbstoffen unter Verwendung des Kondensationsprodukts und die hergestellten Schwefelfarbstoffe

Die Erfindung betrifft ein mindestens 60 Gew.% des Indophenol-S-Oxids I

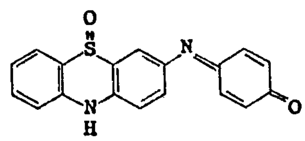

(I)

enthaltendes Kondensationsprodukt aus Phenothiazin und p-Nitrosophenol sowie ein Verfahren zu seiner Herstellung. Außerdem betrifft die Erfindung ein Verfahren zur Herstellung von Schwefelfarbstoffen und die hergestellten Schwefelfarbstoffe.

Nach "Journal of the Chemical Society, London", Band 125, Seiten 802 und 803 (1924) ist es möglich, Phenothiazin in konzentrierter Schwefelsäure mit p-Nitrosophenol zu kondensieren und das erhaltene Kondensationsprodukt bei 250 bis 300° mit Schwefel und Natriumsulfid zu grünen Schwefelfarbstoffen zu verschmelzen. Die in dieser Weise hergestellten Schwefelfarbstoffe zeigen jedoch eine Reihe schwerwiegender Nachteile, insbesondere schlechte Ausbeute, geringe Farbstärke, schlechte Loslichkeit in Alkali-Dithionit und -Sulfidküpen, stumpfe Nuancen, ungleichmäßigen Ausfall der Produktionschargen, geringe Echtheiten, insbesondere der Naßechtheiten und der Peroxid-Waschechtheit, aufgrund deren sie bisher keinen Eingang in die Praxis gefunden haben.

Um grune Schwefelfärbungen auf Baumwolle zu erhalten, war es daher bisher erforderlich, entweder, Schwefel enthaltende Derivate des Kupferphthalocyanis einzusetzen, die nicht mit Natriumdithionit verküpbar sind und deren Einsetzbarkeit daher begrenzt und deren Herstellung technisch verhältnismäßig aufwendig ist, was ihren Einsatz erheblich verteuert, oder aber auf grüne Schwefelfarbstoffe, wie beispielsweise Sulphur Green 2, C.I. Nr. 53 571, oder Sulphur Green 3, C.I. Nr. 53 570, zurückgreifen, die jedoch schlechte Naßechtheiten, insbesondere eine schlechte Peroxid-Waschechtheit haben und daher nicht für Färbungen mit hohem Echtheitsniveau verwendet werden können.

Es bestand daher ein dringendes Bedürfnis nach einem Verfahren, das es gestattet, auf technisch einfache Weise Schwefelfarbstoffe in hoher Reinheit mit guter Löslichkeit, guter Ausbeute und hoher Farbstärke herzustellen, die auf Baumwolle grüne Färbungen liefern.

Überraschenderweise wurde nun gefunden, daß diese Aufgabe gelöst werden kann, wenn solche Kondensationsprodukte aus Phenothiazin und p-Nitrosophenol der Schwefelung in der Back- oder Kochschmelze unterworfen werden, die mindestens 60 Gew.% des Indophenol-S-Oxids der Formel

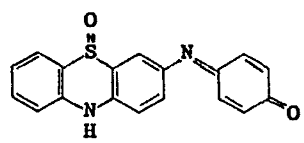

I

enthalten. Derartige Kondensationsprodukte werden nach dem Verfahren der Erfindung hergestellt durch Umsetzung von Phenothiazin und p-Nitrosophenol in Schwefelsäure. Das erfindungsgemäße Verfahren ist dadurch gekennzeichnet, daß das Phenothiazin vor oder während der Umsetzung mit p-Nitrosophenol mit einem zusätzlichen Oxidationsmittel oxidiert wird.

Bei dem bisher bekannten Verfahren, bei dem Phenothiazin in konzentrierter Schwefelsäure mit 1 Mol p-Nitrosophenol umgesetzt wird, wirkt vor allem die als Lösungsmittel verwendete Schwefelsäure als Oxidationsmittel. Bei dem erfindungsgemäßen Verfahren wird dagegen zusätzlich zu der bei dem bekannten Verfahren eingesetzten Schwefelsäure und 1 Mol p-Nitrosophenol ein zusätzliches Oxidationsmittel verwendet.

Als zusätzliche Oxidationsmittel können anorganische oder organische Oxidationsmittel verwendet werden. Geeignete anorganische Oxidationsmittel sind beispielsweise Halogene, wie Chlor oder Brom, Stickoxide, $HNO_2$, $HNO_3$, oder Peroxide und Salze von Persäuren, insbesondere Wasserstoffperoxid. Besonders vorteilhaft ist die Verwendung von Schwefeltrioxid als zusätzliches Oxidationsmittel, das zweckmäßigerweise in Form von rauchender Schwefelsäure (Oleum) mit einem Gehalt von etwa 65 Gew.% $SO_3$ eingesetzt wird. Bei der Verwendung von Schwefeltrioxid wird das Phenothiazin zweckmäßigerweise in 100 %iger Schwefelsäure gelöst.

Geeignete organische Oxidationsmittel sind beispielsweise Peroxicarbonsäuren, wie z.B. Peroxiessigsäure, Chinone, wie Benzochinon, Tetrachlorbenzochinon oder Dicyandichlorbenzochinon, Auch p-Nitrosophenol kann als zusätzliches Oxidationsmittel verwendet werden.

Bei dem erfindungsgemäßen Verfahren werden zur vollständigen Oxidation von 1 Mol Phenothiazin theoretisch ein Oxidationsäquivalent des zusätzlichen Oxidationsmittels benötigt. In der Regel erhält man jedoch bereits mit mindestens 0,5 Oxidationsäquivalenten des zusätzlichen Oxidationsmittels gute

Ergebnisse. Bei dem bevorzugt als zusätzlichem Oxidationsmittel verwendeten Schwefeltrioxid entspricht ein Oxidationsäquivalent 0,5 Molen. Ein Oxidationsäquivalent p-Nitrosophenol entspricht 0,25 Mol. Da p-Nitrosophenol bei dem erfindungsgemäßen Verfahren als zusätzliches Oxidationsmittel und als Kondensationsmittel für das oxidierte Phenothiazin wirkt, werden von p-Nitrosophenol, bezogen auf 1 Mol Phenothiazin, insgesamt mindestens 1,125 Mole benötigt. Es hat sich gezeigt, daß man bei Verwendung von p-Nitrosophenol sehr gute Ergebnisse bei der Verwendung von insgesamt mindestens 1,2 Molen erhält.

Es kann angenommen werden, daß zunächst das Phenothiazin von der Schwefelsäure zu einem Radikalkation oxidiert wird, wobei Wasser entsteht, und daß auch bei der Weiteroxidation des Radikalkations mit dem zusätzlichen Oxidationsmittel zu einem Dikation Wasser gebildet wird. Wenn nun als zusätzliches Oxidationsmittel ein solches verwendet wird, das z.B. Wie Schwefeltrioxid mit Wasser reagiert, muß das Reaktionswasser durch andere Mittel, z.B. durch Zugabe von Phosphorpentoxid, gebunden werden, oder es muß ein entsprechend größerer Überschuß des zusätzlichen Oxidationsmittels verwendet werden. Wenn kein weiteres Mittel zur Bindung des Reaktionswassers zur Anwendung kommt, benötigt man zur vollständigen Oxidation und Bindung des Reaktionswassers 2 Mole Schwefeltrioxid.

Wenn das Phenothiazin nicht in konzentrierter Schwefelsäure gelöst, sondern zu einer Lösung des p-Nitrosophenols in verdünnterer Schwefelsäure gegeben wird, dann bildet sich das Radikalkation durch Reaktion mit der verdünnten $H_2SO_4$ nur langsam, und ein größerer Überschuß an p-Nitrosophenol ist erfoderlich.

Ein gewisser Nachteil des Einsatzes organischer Oxidationsmittel besteht darin, daß die reduzierten Formen dieser Verbindungen oder deren Folgeprodukte sich anschließend in den Reaktionsmischungen wiederfinden und entweder aus den isolierten erfindungsgemäßen Kondensationsprodukten oder aber gegebenenfalls aus den Abwässern entfernt werden müssen. Man wird daher bestrebt sein, bei Anwendung eines Überschusses an zusätzlichem Oxidationsmittel, diesen Überschuß möglichst gering zu halten. Normalerweise wird es nicht erforderlich sein, bei Verwendung eines organischen Oxidationsmittels einen Überschuß von mehr als 4 Oxidationsäquivalenten anzuwenden. Bei Verwendung von p-Nitrosophenol ist es somit normalerweise nicht erforderlich, mehr als insgesamt 2 Mole für zusätzliches Oxidations- und Kondensationsmittel anzuwenden.

Bei der Verwendung des als zusätzlichem Oxidationsmittel bevorzugten Schwefeltrioxids treten auch bei Anwendung eines größeren Überschusses keine Verunreinigungen der Kondensationsprodukte oder zusätzliche Abwasserprobleme auf. Obwohl ein größerer Überschuß an Schwefeltrioxid in keinem Fall schadet, wird es normalerweise nicht erforderlich sein, mehr als 10 Mol Schwefeltrioxid einzusetzen.

Normalerweise führt man die Oxidation des Phenothiazins in einer mindestens 60 %igen, besser jedoch in einer mindestens 80 %igen, vorzugsweise in einer 90 bis 100 %igen Schwefelsäure und zweckmäßigerweise bei Temperaturen von − 40°C bis + 40°C, vorzugsweise bei Temperaturen von 0° bis 25°C, durch. Bei der Oxidation muß normalerweise gekühlt werden.

Nach beendeter Oxidation wird die Kondensation des Oxidationsprodukts mit p-Nitrosophenol erfindungsgemäß in 60 bis 90 %iger, vorzugsweise 75 bis 82 %iger Schwefelsäure vorgenommen. Sofern die Oxidation des Phenothiazins in einer konzentrierteren Schwefelsäure durchgeführt worden ist. wird die Schwefelsäure, beispielsweise durch Zugabe von Eis, entsprechend verdünnt. Die Kondensation erfolgt zweckmäßigerweise bei Temperaturen vcn −20°C bis +20°C, vorzugsweise von −10°C bis +10°C. Die Reaktion ist schwach exotherm, so daß die gewünschte Reaktionstemperatur durch Kühlung eingehalten werden muß. Die Isolierung der erhaltenen Kondensationsprodukte erfolgt in üblicher Weise, beispielsweise durch Eintragen des Reaktionsgemisches in Wasser, Abfiltrieren, Auswaschen, Neutralisieren und Trocknen.

Die Konzentrationsangaben für die Schwefelsäure beziehen sich im Rahmen der vorliegenden Erfindung nur auf die Menge der Komponenten Wasser und $H_2SO_4$ in den Reaktionsgemischen. Bei der Berechnung der Schwefelsäurekonzentration ist auch der Schwefelsäureverbrauch und die Bildung von Reaktionswasser zu berücksichtigen.

Bei der Verwendung von p-Nitrosophenol erfolgen die Oxidationen mit dem zusätzlichen Oxidationsmittel und die Kondensation gleichzeitig. Diese gleichzeitige Oxidation und Kondensation wird zweckmäßigerweise in einer 60 bis 90 %igen, vorzugsweise 75 bis 82 %igen Schwefelsäure durchgeführt. Um die erforderliche Menge des zusätzlichen Oxidationsmittels möglichst gering zu halten, empfiehlt es sich, das Phenothiazin zunächst in konzentrierter Schwefelsäure zu lösen und diese Lösung vor dem Zusatz des p-Nitrosophenol auf eine Konzentration der Schwefelsäure von 60 bis 90%, vorzugsweise 75 bis 82%, zu verdünnen.

Die erfindungsgemäß Anwendung eines zusätzlichen Oxidationsmittels und die Durchführung der Kondensation zwischen dem oxidierten Phenothiazin und dem p-Nitrosophenol in 60 bis 90 %iger, vorzugsweise 75 bis 82 %iger Schwefelsäure führt überraschenderweise in hoher Ausbeute zu Produkten, die nur sehr geringe Anteile von solchen Nebenprodukten enthalten, deren Gegenwart zu einer Qualitätsminderung der erhaltenen Schwefelfarbstoffe führt. So sind die erfindungsgemäß hergestellten Kondensationsprokte praktisch frei von unumgesetztem Phenothiazin, und die Summe der chemisch undefinierten Nebenprodukte liegt unter 20%, in aller Regel sogar unter 10%. Der Hauptbestandteil der

erfindungsgemäß hergestellten Kondensationsprodukte ist das Indophenol-S-Oxid I mit einem Anteil von 60 bis 100 Gew.%, vorzugsweise 80 bis 100 Gew.% und insbesondere 90 bis 100 Gew.%. Außerdem enthalten die erfindungsgemäß hergestellten Kondensationsprodukte das Indophenol Ia mit einem Anteil von 0 bis 40 Gew.%, vorzugsweise 0 bis 20 Gew.% und insbesondere 0 bis 10 Gew.%.

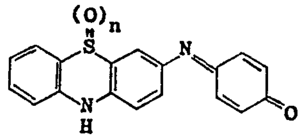

I : n = 1
Ia : n = 0

Kondensationsprodukte aus Phenothiazin und p-Nitrosophenol mit einem so hohen Gehalt an Indophenol-S-Oxid der Form I sind neu und konnten nach bisher bekannten Kondensationsverfahren nicht hergestellt werden. Das Indophenol-S-Oxid der Formel I kann auch ganz oder zum Teil in seinen tautomeren Formen vorliegen.

Die erfindungsgemäß hergestellten Kondensationsprodukte werden durch Schwefelung in der Back- oder Kochschmelze in an sich bekannter Weise in Schwefelfarbstoffe, überführt, die auf Baumwolle grüne und auf Polyamid grüne bis schwarze Färbungen liefern. Diese Schwefelungsverfahren sind beispielsweise in "Venkataraman, The Chemistry of Synthetic Dyes, Bd. 2 (1952), Seite 1962 ff. und Seite 1103 ff. sowie Bd. 7 (1974), Seite 24 ff., Academic Press, New York, San Francisco, London", beschrieben.

Vorzugsweise erfolgt die Schwefelung der erfindungsgemäßen Kondensationsprodukte nach dem Verfahren der Kochschmelze.

Besonders geeignet sind Kochschmelzen, die unter Verwendung von Alkalipolysulfid arbeiten, wobei pro Mol des zu schwefelnden Produkts 2 bis 6 Mole, vorzugsweise 2,5 bis 4 Mole Natriumsulfid und 7 bis 30 Mole, vorzugsweise 13 bis 18 Mole Schwefel eingesetzt werden. Sie werden in den für Schwefelschmelzen üblichen Lösungsmitteln durchgeführt. Als Lösungsmittel, in denen zweckmäßigerweise gearbeitet werden kann, sind Alkanole oder Cycloalkanole mit 1 bis 7 C-Atomen, wie z.B. Methanol, Äthanol, Propanol, Isopropanol, n-Butanol, Isobutanol, Amylalkohol, Cyclohexanol, Methylcyclohexanol und Monoalkyläther insbesondere Monoalkyläther von Äthylenglykol und Diäthylenglykol wie z.B. Äthylenglykol-monomethyläther, -mono-äthyläther, -monopropyläther, Diäthylenglykolmono-methyläther, -mono-äthyläther und -mono-propyläther geeignet.

Die Schwefelung der Kondensationsprodukte kann unter Ausschluß von Wasser oder in rein wäßrigem Medium erfolgen, wird aber normalerweise in wasserhaltigen Lösungsmitteln mit einem Wassergehalt von 5 bis 70% durchgeführt. Im Falle der mit Wasser mischbaren Lösungsmittel arbeitet man vorzugsweise unter Verwendung der üblichen hydrotropen Verbindungen, z.B. des Natriumsalzes der Xylolsulfonsäure.

Die Kochschmelze erfolgt bei Temperaturen von 80 bis 300°C, vorzugsweise 100 bis 180°C, insbesondere im Bereich von 105 bis 130°C und zweckmäßigerweise bei einer Reaktionsdauer von 1 bis 100 vorzugsweise von 20 bis 80 Stunden.

Bei der Schwefelung der erfindungsgemäß hergestellten Kondensationsprodukte durch Backschmelze werden diese mit der 7 bis 30-fachen Molmenge Schwefel und der 2 bis 6-fachen Molmenge Natriumsulfid 1 bis 20 Stunden auf 200 bis 300°C erhitzt. Auch hierbei kann zunächst im Lösungsmittel, vorzugsweise in Wasser, gearbeitet werden, um eine gute Homogenität des Reaktionsansatzes zu gewährleisten. Das Lösungsmittel wird dann abgedampft und die lösungsmittelfreie Schmelze zweckmäßigerweise in Schutzgasatmosphäre auf die gewünschte Temperatur erwärmt.

Werden anstelle der rohen, erfindungsgemäß hergestellten Phenothiazin-p-Nitrosophenol-Kondensationsprodukte die daraus isolierten Verbindungen I und Ia in der oben angegebenen, an sich bekannten Weise geschwefelt, so gelangt man ebenfalls zu erfindungsgemäßen grünen Schwefelfarbstoffen. Es ist auch möglich, die erfindungsgemäßen grünen Schwefelfarbstoffe herzustellen durch Schwefelung der Leukoverbindung des Indophenols I der Formel

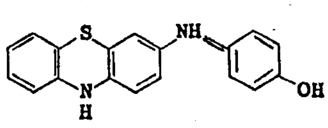

oder von rohen Reduktionsprodukten der erfindungsgemäß hergestellten Kondensationsprodukte, die überwiegend diese Leukoverbindung enthalten.

Die Reduktion der Kondensationsprodukte kann mit den üblichen Reduktionsmitteln in an sich bekannter Weise, beispielsweise mit Natriumhydrogensulfid bei 50—80°C, durchgeführt werden. Es ist anzunehmen, daß auch bei der Schwefelung erfindungsgemäßen Kondensationsprodukte zumindest ein Teil der Kondensationsprodukte zunächst reduziert wird und anschließend erst Schwefel aufnimmt.

Die nach dem erfindungsgemäßen Verfahren hergestellten Schwefelfarbstoffe können auf übliche Weise isoliert und danach in die verschiedenen Handelsformen gebracht werden.

Wurde die Schwefelung in einem mit Wasser mischbaren Lösungsmittel in Gegenwart von hydrotropen Verbindungen ausgeführt, so kann die Schmelze auch ohne Isolierung des Farbstoffs direkt zu flüssigen gebrauchsfertigen Farbstoffen weiterverarbeitet werden.

Die erfindungsgemäß herstellbaren Schwefelfarbstoffe lassen sich durch Reduktionsmittel, wie Natriumdithionit oder Alkalisulfid sehr gut reduzieren und ergeben so klare wässrige Lösungen. Sie können zum Färben von pflanzlichen Fasern und von Polyamid nach den üblichen für das Färben mit Schwefel- oder Schwefelküpenfarbstoffen bekannten Verfahren eingesetzt werden.

Hierzu werden sie mit Reduktionsmitteln, wie z.B. Natriumformaldehyd-sulfoxylat, Glukose oder vorzugsweise Natrium-dithionit, Natriumsulfid oder Natriumhydrogensulfid, in die lösliche Leukoform überführt, die auf die Fasern aufzieht. Die in flüssiger gebrauchsfertiger Form vorliegenden Farbstoffe enthalten die lösliche Leukoform und Reduktionsmittel und können auch ohne weiteren Zusatz von Reduktionsmitteln zum Färben der genannten Fasern verwendet werden.

Nach dem Aufziehen der Farbstoffe auf die Fasern wird die Leukoform der erfindungsgemäßen Schwefelfarbstoffe in üblicher Weise, beispielsweise durch "Verhängen" der Färbungen an der Luft oder Oxidation mit Oxidationsmitteln, wie beispielsweise Wasserstoffperoxid, Alkalichlorit oder Alkalijodat wieder in die unlösliche Form des Schwefelfarbstoffs überführt. Die erfindungsgemäß herstellbaren Schwefelfarbstoffe färben die Zellulose in vollen grünen Tönen.

Auf Zellulosefasern haben die erfindungsgemäßen Farbstoffe gegenüber den im Farbton nächstliegenden Handelsprodukten Sulphur Green 2, C.I. Nr. 53 571, und Sulphur Green 3, C.I. Nr. 53 570, insbesondere folgende Vorteile; sehr gute Lichtechtheit, sehr gute Naßechtheiten, sehr gute Peroxidwaschechtheit nach DIN 54 015 und gute Mercerisierechtheit.

Auf Polyamid erhält man je nach Reduktionsmittel und eingesetzter Farbstoffmenge grüne bis schwarze Farbtöne. Durch Einsatz einer ausreichenden Farbstoffmenge — in der Regel 3 bis 4%, bezogen auf Fasergewicht — unter Verwendung von Natriumhydrogensulfid als Reaktionsmittel kann man auf Polyamid schwarze Farbtöne mit sehr guten Echtheitseigenschaften erhalten. Die Färbungen haben insbesondere eine sehr gute Lichtechtheit und sehr gute Naßechtheiten. Gegenüber dem Handelsprodukt Color-Index Sulphur Black 11, C.I. Nr. 53 290, besitzen die neuen Farbstoffe auf Polyamid unter anderem den Vorteil der leichteren Oxidierbarkeit, so erhält man durch Oxidation mit Wasserstoffperoxid einwandfreie schwarze Färbungen ohne Neigung zum Bronzieren.

Ein weiterer Vorteil der erfindungsgemäßen Farbstoffe ist ihre Stabilität gegen die Einwirkung von Alkalidithionit — und Alkali-Formaldehyd-sulfoxylat — sowie ihre einfache technische Herstellbarkeit.

Gegenüber den nach den sehr allgemeinen Angaben des "Journal of the Chemical Society, London" 125, Seiten 802 und 803, hergestellten Schwefelfarbstoffen zeichnen sich die erfindungsgemäßen Farbstoffe durch wesentlich bessere Echtheitseigenschaften, insbesondere durch bessere Naßechtheiten und bessere Peroxid-Waschechtheit durch klarere Nuancen, wesentlich bessere Löslichkeit in Alkali-Dithionit- und-Sulfidküpen, gleichmäßigeren Ausfall der Produktionschargen, größbere Farbstärke, bessere Ziehvermögen und wesentlich höhere Farbausbeute aus.

In den folgenden Ausführungsbeispielen sind "Teile"Gewichtsteile und alle %-Angaben Gewichtsprozente.

Beispiel 1

40 Teile Phenothiazin werden in 160 Teilen 100%-iger Schwefelsäure unter Kühlung bei Raumtemperatur gelöst. Dann werden 80 Teile rauchende Schwefelsäure mit einem Gehalt von 62,5 Gew.% freiem $SO_3$ unter Kühlung bei Raumtemperatur zugetropft. Nach einer Stunde Nachrühren werden unter Kühlung bei Raumtemperatur 23 Teile 48%-ige Schwefelsäure zugetropft und 38 Teile Eis zugegeben. Man kühlt die Lösung auf —5°C und fügt unter Kühlung und kräftigem Rühren 30,5 Teile wasserhaltige 85%-ige p-Nirosophenolpaste hinzu. Das Reaktionsgemisch wird vier Stunden unter Kühlung bei 0°C nachgerührt. Man gießt anschließend in ein kräftig gerührtes Gemisch aus 200 Teilen Wasser und 400 Teilen Eis. Das Produkt wird abgesaugt, mit ca. 500 Teilen Wasser gewaschen, mit Wasser angeschlämmt, mit Natronlauge neutralisiert, abgesaugt, salzfreigewaschen, getrocknet und gemahlen. Man erhält etwa 64 Teile eines Kondensationsproduktes mit einem Gehalt von etwa 93 Gew.% der Verbindung der Formel I (Bestimmung des Gehaltes durch potentiometrische Titration mit Titantrichlorid) und weniger als 0,5 Gew.% Phenothiazin.

Analyse, ber. für das Indophenol I: ($C_{18}H_{12}N_2O_2S$)

| | | | | | |
|---|---|---|---|---|---|
| ber.: | C:67,5% | H:3,75% | N:8,75% | 0:10% | S:10 % |
| gef.: | C:66,8% | H:3,6 % | N:8,4 % | 0:11% | S:10,3% |

Das IR-Spektrum weist charakteristische Absorptionsbanden bei ca. 1345 $cm^{-1}$, 735 $cm^{-1}$ und bei ca. 1125 $cm^{-1}$ auf.

Das Produkt löst sich in Dimethylformamid und Tetramethylensulfon mit roter Farbe. Die äthanolische Lösung hat im sichtbaren Bereich ein Absorptionsmaximum bei 530 nm.

Das gleiche Produkt wie oben erhält man, wenn man die rauchende Schwefelsäure zusammen mit der 100%-igen Schwefelsäure vorlegt das Phenothiazin darin unter Kühlung bei 20°—25°C einträgt und im übrigen nach den Angaben dieses Beispiels weiter arbeitet.

Beispiel 2

40 Teile Phenothiazin werden in 250 Teilen konzentrierter Schwefelsäure unter Kühlung bei Raumtemperatur gelöst. Man gibt unter Kühlung bei Raumtemperatur 47 Teile Eis zu. Dann kühlt man die Lösung auf −5°C und fügt unter Kühlung und kräftigem Rühren 36,5 Teile wasserhaltige 85%-ige p-Nitrosophenolpaste hinzu. Das Reaktionsgemisch wird vier Stunden unter Kühlung bei 0°C nachgerührt. Man gießt anschließend in ein kräftige gerührtes Gemisch aus 1000 Teilen Wasser und 700 Teilen Eis. Das Produkt wird abgesaugt, mit ca. 500 Teilen Wasser gewaschen, mit Wasser angeschlämmt, mit Natronlauge neutralisiert, abgesaugt, salzfrei gewaschen, getrocknet und gemahlen. Man erhält etwa 61 Teile eines Kondensationsproduktes mit einem Gehalt von etwa 93 Gew.% der Verbindung I (Bestimmung des Gehaltes durch potentiometrische Titration mit Titantrichlorid) und etwa 2 Gew.% Phenothiazin.

Analyse, be. für das Indophenol I: ($C_{18}H_{12}N_2O_2S$)

| | | | | | |
|---|---|---|---|---|---|
| ber.: | C:67,5% | H:3,75% | N:8,75% | 0:10% | S:10 % |
| gef.: | C:67,3% | H:3,7 % | N:8,8 % | | S:10,3% |

Das IR-Spektrum, das Absorptionsspektrum im sichtbaren Bereich und die Lösungsfarbe stimmen mit dem Produkt von Beispiel 1 überein.

Beispiel 3
(Vergleichsbeispiel zu Beispiel 2)

40 Teile Phenothiazin werden in 250 Teilen konzentrierter Schwefelsäure unter Kühlung bei Raumtemperatur gelöst. Man kühlt die Lösung auf −5°C und fügt unter Kühlung und kräftigem Rühren 30,5 Teile wasserhaltige 85%-ige p-Nitrosophenolpaste hinzu. Das Reaktionsgemisch wird vier Stunden unter Kühlung bei 0°C nachgerührt. Man gießt anschliesend in ein kräftig gerührtes Gemisch aus 1000 Teilen Wasser und 700 Teilen Eis. Das Produkt wird abgesaugt, mit ca. 500 Teilen Wasser gewaschen, mit Wasser angeschlämmt, mit Natronlauge neutralisiert, abgesaugt, salzfrei gewaschen, getrocknet und gemahlen. Man erhält etwa 52 Teile eines Produktes mit einem Gehalt von ca. 4 Gew.% Phenothiazin. Nach der Bestimmungsmethode durch Titration mit $TiCl_3$ erhält man ungefährt folgende Werte: ca. 35 Gew.% für die Verbindung der Formel I und ca. 40 Gew.% für die Verbindung der Formel Ia. Zu ca. 20% besteht das erhaltene Produkt aus undefinierten organischen Verbindungen.

Beispiel 4

43,5 Teile wasserhaltige 85%-ige p-Nitrosophenolpaste werden unter Kühlung in 300 Teile 80%-ige Schwefelsäure unter 0°C eingetragen und gelöst. In diese Lösung werden unter Kühlung 40 Teile gepulfertes Phenothiazin bei −10°C bis 0°C unter kräftigem Rühren eingestreut und gleichmäßig verrührt. Das Reaktionsgemisch wird vier Stunden uner Kühlung bei 0°C nachgerührt. Dann gießt man das Reaktionsgemisch in ein kräftig gerührtes Gemisch aus 1000 Teilen Wasser und 700 Teilen Eis. Das Produkt wird abgesaugt, mit ca. 500 Teilen Wasser gewaschen, mit Wasser angeschlämmt, mit Natronlauge neutralisiert, abgesaugt, salzfrei gewaschen, getrocknet und gemahlen. Man erhält etwa 64 Teile eines Kondensationsproduktes mit einem Gehalt von etwa 85 Gew.% der Verbindung der Formel I (Bestimmung des Gehaltes durch potentiometrische Titration mit Titantrichlorid) und etwa 0,5 Gew.% Phenothiazin.

Analyse, ber. für das Indophenol I: ($C_{18}H_{12}N_2O_2S$)

| | | | | | |
|---|---|---|---|---|---|
| ber.: | C:67,5% | H:3,75% | N:8,75% | 0:10 % | S:10 % |
| gef.: | C:66,8% | H:3,5 % | N:8,5 % | 0:10,6% | S:10,3% |

Das IR-Spektrum, das Absorptionsspektrum im sichtbaren Bereich und die Lösungsfarbe stimmen mit dem Produkt vom Beispiel 1 überein.

Beispiel 5
(Vergleichsbeispiel zu Beispiel 4)

30,5 Teile wasserhaltige 85%-ige p-Nitrosophenolpaste werden unter Kühlung in 250 Teile konzentrierte Schwefelsäure unter 0°C eingetragen und gelöst. In diese Lösung werden unter Kühlung 40 Teile gepulvertes Phenothiazin bei −10°C bis 0°C unter kräftigem Rühren eingestreut und gleichmäßig verrührt. Das Reaktionsgemisch wird vier Stunden unter Kühlung bei 0°C nachgerührt. Dann gießt man das Reaktionsgemisch in ein kräftig gerührtes Gemisch aus 1000 Teilen Wasser und 700 Teilen Eis. Das Produkt wird abgesaugt, mit ca. 500 Teilen Wasser gewaschen, mit Wasser angeschlämmt, mit Natronlauge neutralisiert, abgesaugt, salzfrei gewashen, getrocknet und gemahlen. Man erhält etwa 50 Teile eines Produktes mit einem Gehalt von ca. 4 Gew.% Phenothiazin. Nach der Bestimmungsmethode durch Titration mit $TiCl_3$ erhält man ungefähr folgende Werte: ca. 35 Gew.% für die Verbindung der Formel I und ca. 35 Gew.% für die Verbindung der Formel Ia.

Beispiel 6

40 Teile des Produktes von Beispiel 1 werden in 800 Teile einer etwa 70°C warmen 10%-igen wässrigen Lösung von Natriumhydrogensulfid eingerührt. Dann wird drei Stunden bei 70°C kräftig nachgerührt, auf Raumtemperatur abgekühlt und das helle reduzierte Produkt abgesaugt und gewaschen. Der Filterkuchen wird mit Wasser angeschlämmt, mit Salzsäure schwach sauer gestellt, abgesaugt, gewaschen und getrocknet. Man erhält etwa 33 Teile der Leukoverbindung.

Analyse, berechnet für die Leukoverbindung des Indophenols I: ($C_{18}H_{14}N_2OS$)

| | | | | | |
|---|---|---|---|---|---|
| ber.: | C:70,59% | H:4,57% | N:9,15% | 0:5,23% | S:10,46% |
| gef.: | C:69,5 % | H:4,4 % | N:9,1 % | 0:6,0 % | S:10,9 % |

Das Produkt is sowohl in alkalischen wässrigen Lösungen von Natriumdithionit als auch in wässrigen Lösungen von Natriumdithionit als auch in wässrigen Lösungen von Natriumsulfid und Natriumhydrogensulfid schwer löslich. Das IR-Specktrum zeigt charakteristische Absorptionsbanden bei 1600, 1495, 1465, 1300, 1235 und 735 $cm^{-1}$. Die den beiden Verbindungen der Formeln I und Ia gemeinsame Absorptionsbande bei 763 $cm^{-1}$ sowie die Absorptionsbanden der Verbindung der Formel I bei 1125 und 1345 $cm^{-1}$ fehlen.

Beispiel 7

44 Teile des Produktes von Beispiel 1 werden in eine Polysulfidlösung von etwa 70°C eingebracht, die aus 205 Teilen 95%-igem n-Butanol, 49 Teilen schuppenförmigem Natriumsulfid (60%-ig) und 58 Teilen Schwefel bereitet wurde. Man erhitzt zum Sieden und hält die Mischung 72 Stunden unter kräftigem Sieden und Rühren am Rückfluß. Man gibt ohne abzukühlen 2,5 Teile Natriumnitrit und 25 Teile 95%-iges n-Butanol hinzu rührt weitere zwei Stunden am Rückfluß. Anschließend fügt man 300 Teile 24°Bé Salzwasser hinzu und destilliert das Butanol mit Wasserdampf ab. Der Farbstoff wird abgesaugt und mit 12°Bé Salzwasser frei von Polysulfid gewaschen Der Filterkuchen wird in heißem Wasser angeschlämmt, mit Salzsäure auf pH — 2 gestellt und eine Stunde bei 60°C nachgerührt. Anschließend wird abgesaugt, neutral gewaschen, getrocknet und gemahlen. Man erhält etwa 61 Teile Farbstoff in Pulverform. Er färbt die Cellulosefaser aus Alkalisulfidoder alkal. Dithionitlösung in grünen Tönen von sehr guter Naßund Lichtechtheit und Polyamid in grünen bis tiefschwarzen Tönen von sehr guter Naß- und Lichtechtheit.

Der Farbstoff ist in Dithionit-, Sulfid- und Hydrogensulfid- küpen ausgezeichnet löslich.

Verwendet man anstelle des Produktes von Beispiel 1 44 Teile des Produktes von Beispiel 6, so erhält man einen Farbstoff mit praktisch gleichen Eigenschaften.

Verwendet man anstelle des Produktes von Beispiel 1 44 Teile der Produkte von Beispiel 2 oder Beispiel 4, so erhält man Farbstoffe mit praktisch gleichen Eigenschaften, jedoch etwas geringerer Löslichkeit.

Beispiel 8

(Vergleichsbeispiel zu Beispiel 7)

Verwendet man in dem Beispiel 7 anstelle des Produktes von Beispiel 1 44 Teile des Produktes von Beispiel 3 und arbeitet man im übrigen nach den Angaben von Beispiel 7, so erhält man etwa 60 Teile Farbstoff in Pulverform. Der Farbstoff unterscheidet sich von dem Farbstoff von Beispiel 7 durch die wesentlich geringere Farbstärke, die deutlich stumpfere Nuance und die wesentlich schlechtere Löslichkeit in Dithionit-, Sulfid- und Hydrogensulfidküpen.

Beispiel 9

(Vergleichsbeispiel zu Beispiel 7)

Verwendet man im Beispiel 7 anstelle des Produktes von Beispiel 1 44 Teile des Produktes von Beispiel 5 und arbeitet man im übrigen nach den Angaben von Beispiel 7, so erhält man etwa 60 Teile Farbstoff in Pulverform. Der Farbstoff unterscheidet sich von dem Farbstoff von Beispiel 7 durch die wesentlich geringere Farbstärke, die deutlich stumpfere Nuance und die wesentlich schlechtere Löslichkeit in Dithionit-, Sulfid- und Hydrogensulfidküpen.

Beispiel 10

44 Teile des Produktes von Beispiel 1 werden in eine Polysulfidlösung von etwa 70°C eingebracht, die aus 50 Teilen Wasser, 50 Teilen Diäthylenglykolmonoäthyläther, 30 Teilen des Natriumsalzes von m-Xylolsulfonsäure, 49 Teilen schuppenformigem Natriumsulfid (60%ig) und 58 Teilen Schwefel bereitet wurde. Man erhitzt zum Sieden und hält die Mischung 72 Stunden unter kräftigem Sieden und Rühren am Rückfluß. Die Schmelzmasse wird darauf durch Zugabe von 42 Teilen des Natriumsalzes von m-Xylolsulfonsäure, 160 Teilen einer 30%-igen Natriumhydrogensulfidlösung, 53 Teilen schuppenformigem Natriumsulfid und 250 Teilen Wasser in Lösung gebracht. Man erhält eine wasserdünne, klare, nicht absetzende, haltbare Lösung des reduzierten Schwefelfarbstoffes, die mit Wasser ohne Ausfällung mischbar ist. Man erhält mit dieser gebrauchsfertigen Farbstofflösung ohne weiteren Zusatz von Reduktionsmitteln auf der Zellulosefaser grüne Färbungen von sehr guter Naß- und Lichtechtheit, auf Polyamidfasern grüne bis tiefschwarze Färbungen von guter

Naß- und Lichtechtheit. Der Farbstoff eignet sich in dieser Form besonders gut für die Anwendung nach kontinuierlich arbeitenden Färbeverfahren.

Verwendet man anstelle des Produktes von Beispiel 1 44 Teile der Produkte von Beispiel 2, 4 oder 6, so erhält man Farbstoffe mit praktisch gleichen Eigenschaften.

Beispiel 11
(Vergelichsbeispiel zu Beispiel 10)

Verwendet man im Beispiel 10 anstelle des Produktes von Beispiel 1 44 Teil des Produktes von Beispiel 3 und arbeitet man im übrigen nach den Angaben von Beispiel 10, so erhält man einen Farbstoff, der sich von dem Farbstoff von Beispiel 10 durch die wesentlich geringere Farbstärke, die erheblich stumpfere Nuance und die wesentlich schlechtere Löslichkeit unterscheidet.

Beispiel 12
(Vergleichsbeispiel zu Beispiel 10)

Verwendet man in dem Beispiel 10 anstelle des Produktes von Beispiel 1 44 Teile des Prokuktes von Beispiel 5 und arbeitet man im übrigen nach den Angaben von Beispiel 10, so erhält man einen Farbstoff, der sich von dem Farbstoff von Beispiel 10 durch die wesentlich geringere Farbstärke, die deutlich stumpfere Nuance und die wesentlich schlechtere Löslichkeit unterscheidet.

Beispiel 13
(Vergleichsbeispiel zu den Beispielen 7 und 10)

Aus 49 Teilen schuppenformigem Natriumsulfid, 58 Teilen Schwefel und 170 Teilen Wasser bereitet man eine Polysulfidlösung. In diese Lösung rührt man in der Wärme 44 Teile des Produktes von Beispiel 3 ein und dampft anschließend das Reaktionsgemisch unter Rühren ein. Nachdem das Wasser verdampft ist, erhitzt man, das Reaktionsgemisch innerhalb von vier Stunden auf eine Temperatur von 250°C und hält es anschließend noch 4 Stunden bei dieser Temperatur. Nach dem Abkühlen wird das Produkt gemahlen. Man erhält etwa 110 Teile Farbstoff. Dieser Farbstoff unterscheidet sich von den Farbstoffen der Beispiele 7 und 10 durch die ganz erheblich gelbere Nuance (oliv), die wesentlich geringere Farbstärke, die schlechtere Löslichkeit und vor allem durch wesentlich schlechtere Naßechtheiten, insbesondere durch die wesentlich schlechtere Peroxid-Waschechtheit.

Beispiel 14

In einem Färbebecher, der sich in einem beheizbaren Bad befindet, verrührt man 0,2 Teile des nach dem Beispiel 7 hergestellten Farbstoffes mit 0,05 Teilen eines anionaktiven Netzmittels vom Typ Nekal BX und 3 Teilen 18%-ige Natronlauge, gibt dann 200 Teile Wasser von 70°C und 0,8 Teile Natriumdithionit dazu und rührt die Küpe, bis der Farbstoff klar gelöst ist. In der fertigen Färbeflotte hält man 10 Teile Baumwollmaterial bei 60°C 45 Minuten ständig in Bewegung. Das gefärbte Baumwollmaterial wird danach der Flotte entnommen und die noch anhaftende Flotte abgequetscht. Anschließend spült man das gefärbte Material gründlich mit kaltem Wasser. Dann wird das gefärbte Material sofort in eine 40°C warme Lösung von 0,23 Teilen einer 35%-igen wässrigen Wasserstoffperoxid-lösung und 1 Teil Natriumdihydrogenphosphat in 200 Teilen Wasser gebracht, die zuvor mit Natronlauge auf pH = 8 eingestellt wurde. Das gefärbte Baumwollmaterial wird 15 Minuten bei 40°C in dem Oxydationsbad in Bewegung gehalten, danach aus dem Bad genommen, gründlich mit kaltem Wasser gespült und bei 100°C getrocknet. Man erhält eine grüne Färbung mit sehr guten Echtheitseigenschaften, insbesondere mit sehr guter Lichtechtheit, sehr guten Naßechtheiten und sehr guter Peroxid-Waschechtheit.

Beispiel 15

In einem Färbebecher, der sich in einem beheizbaren Bad befindet, verrührt man 0,2 Teile des nach dem Beispiel 7 hergestellten Farbstoffes mit 0,05 Teilen eines anionaktiven Netzmittels vom Typ Nekal BX und 8 Teilen einer 10%-igen wässrigen Sodalösung. Man gibt 1,25 Teile einer 20%-igen wässrigen Natriumhydrogensulfidlösung, 0,85 Teile einer 45%-igen wässrigen Natriumtetrasulfidlösung und 100 Teile heißes Wasser hinzu und löst den Farbstoff durch Kochen. Anschließend verdünnt man die Lösung mit 100 Teilen heißes Wasser und fügt 4 Teile Natriumsulfat hinzu. In der fertigen Färbeflotte hält man 10 Teile Baumwollmaterial bei 90°C eine Stunde ständig in Bewegung. Das gefärbte Baumwollmaterial wird danach der Flotte entnommen und die noch anhaftende Flotte abgequetscht. Anschließend spült man das gefärbte Material gründlich mit kaltem Wasser. Dann wird das gefärbte Material sofort in eine 40°C warme Lösung von 0,23 Teilen 35%-iges Wasserstoffperoxid und 1 Teil Natriumdihydrogenphosphat in 200 Teilen Wasser gebracht, die zuvor mit Natronlauge auf pH = 8 eingestellt wurde. Das gefärbte Baumwollmaterial wird 15 Minuten bei 40°C in dem Oxydationsbad in Bewegung gehalten, danach aus dem Bad genommen gründlich mit kaltem Wasser gespült und bei 100°C getrocknet.

Man erhält eine grüne Färbung mit sehr guten Echtheitseigenschaften, insbesondere mit sehr guter Lichtechtheit, sehr guten Naßechtheiten und sehr guter Peroxid-Waschechtheit.

Beispiel 16

In einem Färbebecher, der sich in einem beheizbaren Bad bedfindet, verrührt man 0,4 Teile des nach dem Beispiel 7 hergestellten Farbstoffes mit 0,05 Teilen, eines anionaktiven Netzmittels vom Typ Nekal BX und 10 Teilen einer 10%-igen Sodalösung. Man gibt 2,3 Teile einer 20%-igen wässrigen Natriumhydrogensulfidlösung, 0,85 Teile einer 45%-igen wässrigen Natriumtetrasulfidlösung und 100 Teile heißes Wasser hinzu und löst den Farbstoff durch Kochen. Anschließend verdünnt man die Lösung mit 100 Teilen heißes Wasser und fügt 4 Teile Natriumsulfat hinzu. In der fertigen Färbeflotte halt man 10 Teile Polyamidgarn bei 95°C eine Stunde ständig in Bewegung. Das gefärbte Polyamidgarn wird darauf der Flotte entnommen und mit kaltem Wasser gründlich gespült. Dann wird das gefärbte Material sofort in eine 40°C warme Lösung von 0,45 Teilen 35%-iges Wasserstoffperoxid und 1 Teil Natriumdihydrogenphosphat in 200 Teilen Wasser gebracht, die zuvor mit Natronlauge auf pH = 8 eingestellt wurde. Das gefärbte Polyamidgarn wird 20 Minuten bei 40°C in dem Oxydationsbad in Bewegung gehalten, danach aus dem Bad genommen, gründlich mit kaltem Wasser gespült und getrocknet.

Man erhält eine schwarze Färbung mit sehr guten Echtheitseigenschaften, insbesondere mit sehr guter Lichtechtheit und sehr guten Naßechtheiten.

Führt man die Beispiele 14-16 unter Einsatz des nach Beispiel 10 erhaltenen Schwefelfarbstoffs aus, so erhält man gleich gute Färbungen.

## Patentansprüche

1. Verfahren zur Herstellung eines Kondensationsprodukts aus Phenothiazin und p-Nitrosophenol, welches das Indophenol-S-oxid der Formel I

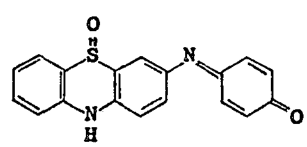

(I)

enthält, durch Umsetzung von Phenothiazin mit p-Nitrosophenol in Schwefelsäure, dadurch gekennzeichnet, daß das Phenothiazin vor oder während der Umsetzung mit einem zusätzlichen Oxidationsmittel oxidiert wird.

2. Verfahren nach Anspruch 1, dadurch gekennzeichnet, däß pro Mol Phenothiazin von dem zusätzlichen Oxidationsmittel mindestens 0,5 Oxidationsäquivalente eingesetzt werden.

3. Verfahren nach Anspruch 1, dadurch gekennzeichnet, daß pro Mol Phenothiazin von dem zusätzlichen Oxidationsmittel mindestens 1 Oxidationsäquivalent eingesetzt wird.

4. Verfahren nach den Ansprüchen 1 bis 3, dadurch gekennzeichnet, daß als zusätzliches Oxidationsmittel Schwefeltrioxid verwendet wird.

5. Verfahren nach den Ansprüchen 1, 3 oder 4, dadurch gekennzeichnet, daß pro Mol Phenothiazin mindestens 0,5 Mole Schwefeltrioxid eingesetzt werden.

6. Verfahren nach den Ansprüchen 1 bis 3, dadurch gekennzeichnet, daß als zusätzliches Oxidationsmittel p-Nitrosophenol verwendet wird.

7. Verfahren nach den Ansprüchen 1, 2 oder 6, dadurch gekennzeichnet, daß pro Mol Phenothiazin für die Kondensation und die zusätzliche Oxidation mindestens 1,125 Mole p-Nitrosophenol eingesetzt werden.

8. Verfahren nach den Ansprüchen 1 bis 3 oder 6, dadurch gekennzeichnet, daß pro Mol Phenothiazin für die Kondensation und die zusätzliche Oxidation mindestens 1,2 Mole p-Nitrosophenol eingesetzt werden.

9. Verfahren nach den Ansprüchen 1 bis 8, dadurch gekennzeichnet, daß die Behandlung mit dem zusätzlichen Oxidationsmittel in 60 bis 100 %iger Schwefelsäure erfolgt.

10. Verfahren nach den Ansprüchen 1 bis 9, dadurch gekennzeichnet, daß die Kondensation des oxidierten Phenothiazin mit p-Nitrosophenol in 60 bis 90 %iger Schwefelsäure erfolgt.

11. Verfahren nach den Ansprüchen 1 bis 10, dadurch gekennzeichnet, daß die Kondensation des oxidierten Phenothiazins mit p-Nitrosophenol in 75 bis 82 %iger Schwefelsäure erfolgt.

12. Kondensationsprodukt aus Phenothiazin und p-Nitrosophenol, dadurch gekennzeichnet, daß es mindestens 60 Gew.% des Indophenol-S-oxids der Formel

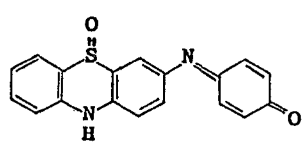

und/oder seiner Tautomeren enthält.

13. Verfahren zur Herstellung von Schwefelfarbstoffen, dadurch gekennzeichnet, daß ein mindestens 60 Gew.% des Indophenol-S-Oxids der Formel

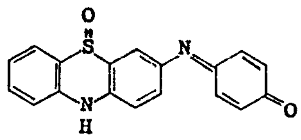

enthaltendes Kondensationsprodukt, das aus Phenothiazin und p-Nitrosophenol in Schwefelsäure unter Anwendung eines zusätzlichen Oxidationsmittels erhalten worden ist oder das aus einem solchen Kondensationsprodukt durch Reduktion erhaltene Reduktionsprodukt, in an sich bekannter Weise in der Back- oder Kochschmelze geschwefelt wird.

14. Die nach dem Verfahren des Anspruch 13 hergestellten Schwefelfarbstoffe.

## Claims

1. A process for preparing a condensation product from phenothiazine and p-nitrosophenol containing the indophenol-S-oxide of formula I

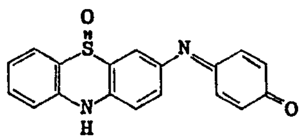

(I)

by reacting phenothiazine with p-nitrosophenol in sulphuric acid, characterised in that the phenothiazine is oxidised with additional oxidising agent before or during the reaction.

2. The process of claim 1, characterised in that per mole of phenothiazine at least 0.5 oxidisation equivalents of the additional oxidising agent are employed.

3. The process of claim 1, characterised in that per mole of phenothiazine at least 1 oxidation equivalent of the additional oxidising agent is employed.

4. The process of claims 1 to 3, characterised in that the additional oxidising agent employed is sulphur trioxide.

5. The process of claims 1, 3 or 4, characterised in that per mole of phenothiazine at least 0.5 mole of sulphur trioxide is employed.

6. The process of claims 1 to 3, characterised in that the additional oxidising agent employed is p-nitrosophenol.

7. The process of claims 1, 2 or 6, characterised in that per mole of phenothiazine at least 1.125 moles of p-nitrosophenol are employed for the condensation and the additional oxidation.

8. The process of claims 1 to 3 or 6, characterised in that per mole of phenothiazine at least 1.2 moles of p-nitrosophenol are employed for the condensation and the additional oxidation.

9. The process of claims 1 to 8, characterised in that the treatment with the additional oxidising agent takes place in 60 to 100% strength sulphuric acid.

10. The process of claims 1 to 9, characterised in that the condensation of the oxidised phenothiazine with p-nitrosophenol takes place in 60 to 90% strength sulphuric acid.

11. The process of claims 1 to 10, characterised in that the condensation of the oxidised phenothiazine with p-nitrosophenol takes place in 75 to 82% strength sulphuric acid.

12. Condensation product from phenothiazine and p-nitrosophenol, characterised in that it contains at least 60% by weight of the indophenol-S-oxide of the formula

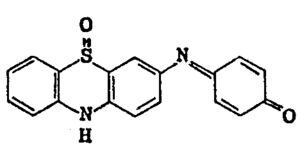

and/or its tautomers.

13. A process for preparing sulphur dyes, characterised in that a condensation product which contains at least 60% by weight of the indophenol-S-oxide of the formula

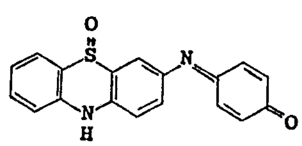

and is obtained from phenothiazine and p-nitrosophenol in sulphuric acid using an additional oxidising agent, or the reduction product obtained from such condensation product by reduction, is thionated in a manner known per se by the bake or reflux process.

14. The sulphur dyes prepared by the process of claim 13.

**Revendications**

1. Procédé de préparation d'un produit de condensation dérivant de la phénothiazine et du p-nitrosophénol, contenant l'indophénol-S-oxyde der formula I:

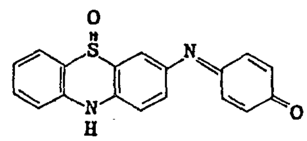

(I)

par réaction de la phénothiazine avec le p-nitroso-phénol dans de l'acide sulfurique, procédé caractérisé en ce qu'on oxyde la phénothiazine, avant ou pendant la réaction, au moyen d'un agent d'oxydation supplémentaire.

2. Procédé selon la revendication 1, caractérisé en ce qu'on utilise au moins 0,5 équivalent d'oxydation de l'agent d'oxydation supplémentaire par mole de phénothiazine.

3. Procédé selon la revendication 1, caractérisé en ce qu'on utilise au moins 1 équivalent d'oxydation de l'agent d'oxydation supplémentaire par mole de phénothiazine.

4. Procédé selon l'une quelconque des revendiactions 1 à 3, caractérisé en ce qu'on utilise l'anhydride sulfurique comme agent d'oxydation supplémentaire.

5. Procédé selon l'une quelconque des revendications 1, 3 et 4, caractérisé en ce qu'on utilise au moins 0,5 mole d'anhydride sulfurique par mole de phénothiazine.

6. Procédé selon l'une quelconque des revendications 1 à 3, caractérisé en ce qu'on utilise le p-nitroso-phénol comme agent d'oxydation supplémentaire.

7. Procédé selon l'une quelconque des revendications 1, 2 et 6, caractérisé en ce qu'on utilise au moins 1,125 mole de p-nitroso-phénol par mole de phénothiazine pour la condensation et l'oxydation supplémentaire.

8. Procédé selon l'une quelconque des revendications 1 à 3 et 6, caractérisé en ce qu'on utilise au moins 1,2 mole de p-nitroso-phénol par mole de phénothiazine pour la condensation et l'oxydation supplémentaire.

9. Procédé selon l'une quelconque des revendications 1 à 8, caractérisé en ce qu'on effectue le traitement par l'agent d'oxydation supplémentaire dans de l'acide sulfurique d'une concentration comprise entre 60 et 100%.

10. Procédé selon l'une quelconque des revendications 1 à 8 9, caractérisé en ce qu'on effectue la condensation de la phénothiazine oxydée avec le p-nitroso-phénol dans de l'acide sulfurique d'une concentration comprise entre 60 et 90%.

11. Procédé selon l'une quelconque des revendications 1 à 10, caractérisé en qu'on effectue la condensation de la phénothiazine oxydée avec le p-nitroso-phénol dans de l'acide sulfurique d'une concentration comprise entre 75 et 82%.

12. Produit de condensation de la phénothiazine et du p-nitroso-phénol, caractérisé en ce qu'il contient au moins 60% en poids de l'indophénol-S-oxyde de formule:

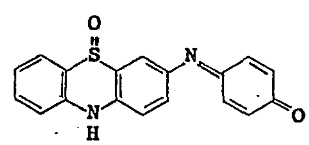

et/ou de ses tautoméres.

13. Procédé de préparation de colorants au soufre, caractérisé en ce qu'on soumet à la sulfuration de manière connue, par fusion à sec ou fusion au bouillon, un produit de condensation qui contient au moins 60% en poids de l'indophénol-S-oxyde de formule:

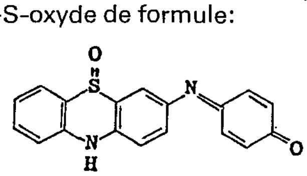

et qui a été obtenu à partie de la phénothiazine et du p-nitroso-phénol dans de l'acide sulfurique avec emploi d'un agent d'oxydation supplémentaire, ou le produit de réduction obtenu à partir d'un tel produit de condensation par réduction.

14. Colorants au soufre qui ont été préparés par le procédé de la revendication 13.